# EUROPEAN PATENT SPECIFICATION

(11) **EP 2 661 257 B1**
(45) Date of publication and mention of the grant of the patent: **15.11.2017**
(21) Application number: 12706345.1
(22) Date of filing: 06.01.2012
(51) Int. Cl.: A61K 9/16, A61K 9/20, A61K 31/663, A61K 9/46

(54) **EFFERVESCENT BISPHOSPHONATE FORMULATIONS**
BISPHOSPHONAT-BRAUSEFORMULIERUNGEN
FORMULATIONS EFFERVESCENTES DE BISPHOSPHONATE

(30) Priority: 06.01.2011 TR 201100153
(43) Date of publication of application: 13.11.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2012/000009
(87) International publication number: WO 2012/093978

(56) References cited:
- WO-A1-2010/090613

## Description

### Technical Field

The present invention relates to highly bioavailable effervescent formulations comprising alendronate and/or a pharmaceutically suitable derivative thereof which protect epithelial and mucosal layer of the mouth, buccal cavity, pharynx, larynx and esophagus from erosion, ulcer and similar irritations in order to be used in prevention and treatment of bone loss and diseases associated with bone loss.

### The Prior Art

Bone loss or in other words "osteoporosis" is defined as a disease characterized by low bone mass and susceptibility to bone fragility as a result of distortion in microstructure of bone tissue and increased risk in bone fracture. The disease does not result in death; however, it appears as a major health problem affecting quality of life in the world.

Bisphosphonates is a group of drugs which is commonly used in prevention and treatment of bone loss and diseases associated with bone loss. The studies conducted have shown that starting bisphosphonate treatment reduces risk of bone fracture when there appears osteoporosis or nontraumatic bone fracture in postmenopausal period *(Fink HA the Fracture Intervention Trial (FIT) 2003).* Alendronate, clodronate, etidronate, ibandronate, pamidronate, risedronate and zoledronate are commonly used bisphosphonates today.

Alendronic acid *(Formula I)* is a bisphosphonate having the chemical name of (4-amino-1-hydroxybutylidene) bisphosphonic acid.

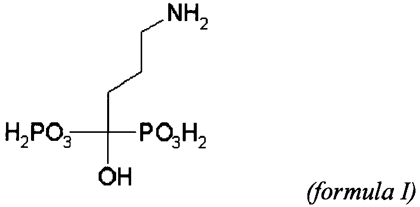

Alendronic acid was first disclosed in the patent numbered US4705651. Furthermore, processes in order to prepare alendronic acid and use of alendronic acid in treatment of osteopathy have been disclosed in said patent.

Alendronate was approved by the FDA for treatment of postmenopausal osteoporosis. Tablet formulation on the market should be taken with a glass of water (200ml) at least half an hour before the first meal of the day in the morning. If the patient takes the drug with less amount of water or does not remain in upright position for half an hour after the intake of the drug, an irritation occurs in upper gastrointestinal system. These gastrointestinal irritations can be specified as erosion, ulcer and similar irritations of mucosal and epithelial layer of the mouth, buccal cavity, pharynx, larynx and esophagus.

Another problem encountered in oral administration of bisphosphonates is that sufficient bioavailability cannot be ensured depending on low absorption in gastrointestinal system. For instance, only 0.5% to 5% of the drug is absorbed and only 20% to 50% of the drug which is absorbed shows efficiency by binding to bone tissue in tablet form. Another factor that affects bioavailability of bisphosphonates is derived from high gastric acid. Therefore, beverages such as coffee, tea, orange juice reduce bioavailability.

Relevant prior art is also WO 2010/090613 A1. When the prior art is taken into consideration, there is need for development of formulations which have higher bioavailability as compared to existing formulations and do not cause erosion, ulcer and similar irritations in gastrointestinal system. The purpose of the formulation of the present invention is to provide highly bioavailable effervescent formulations which do not cause erosion, ulcer and similar irritations in gastrointestinal system.

### Description of the Invention

The present invention relates to an effervescent formulation comprising alendronate, a pharmaceutically acceptable diluent and optionally at least one other excipient, characterized in that the diluent used in the formulation is maltodextrin and the ratio of alendronate to maltodextrin used in the formulation is in the range of 1.1 to 0.9 by weight. The inventors have surprisingly found that effervescent formulations wherein the ratio of alendronate:maltodextrin is in the range of 1:1 to 0.9 by weight do not cause erosion, ulcer and similar irritations in gastrointestinal system and they have higher bioavailability than existing formulations.

Alendronate comprised in the effervescent formulation of the present invention can be in the form of its pharmaceutically acceptable hydrates, solvates, esters, enantiomers, polymorphs, crystalline forms, amorphous forms, salts or free base form and/or a combination thereof.

Alendronate comprised in the effervescent formulation of the present invention is preferably in salt form.

These salts can be selected from organic acid salts such as hydrochloride, hydrobromide, sulphate, phosphate, formate, acetate, trifluoroacetate, methanesulphonate, benzenesulphonate, toluenesulfonate; alkaline metal salts such as sodium, potassium; alkaline earth salts such as calcium, magnesium; organic amine salts such as trimethylamine, triethylamine, pyridine, picoline, dicyclohexylamine or a combination thereof.

Alendronate comprised in the effervescent formulation of the present invention is in alendronate sodium form.

Alendronate sodium comprised in the effervescent formulation of the present invention is preferably in hydrate form.

These hydrates can be selected from hydrates such as monohydrate, dihydrate, trihydrate, hemihydrate, ¼ hydrate, ⅓ hydrate, ²/₃ hydrate, ¾ hydrate, ⁵/₄ hydrate, ⁴/₃ hydrate, ⁵/₂ hydrate and ³/₂ hydrate.

Alendronate sodium comprised in the effervescent formulation of the present invention is preferably in trihydrate form.

Alendronate comprised in the effervescent formulation of the present invention is alendronate sodium trihydrate.

The amount of alendronate comprised in the effervescent formulation of the present invention is in the range of 1-200 mg, preferably in the range of 5-100 mg.

Alendronate comprised in the effervescent formulation of the present invention is in the range of 0.1-30% by weight, preferably in the range of 1-20% by weight.

The effervescent formulation of the present invention comprises at least one pharmaceutically acceptable effervescent couple in addition to alendronate and diluent.

In other words, one characteristic feature of the effervescent formulations of the present invention is that
- the formulations comprise alendronate, maltodextrin, at least one effervescent couple and optionally at least one other pharmaceutically acceptable excipient and
- alendronate: maltodextrin, ratio is in the range of 1.1 to 0.9 by weight.

The term "effervescent couple" refers to use of an acidic agent and a basic agent together. The effervescent couple comprised in the effervescent formulation of the present invention is in the range of 50-90% by weight.

In other words, one characteristic feature of the effervescent formulations of the present invention is that
- the formulations comprise alendronate in the range of 0.1 to 30 % by weight, maltodextrin, at least one effervescent couple in the range of 50 to 90 % by weight and optionally at least one other pharmaceutically acceptable excipient and
- alendronate :maltodextrin ratio is in the range of 1.1 to 0.9 by weight.

The pharmaceutically acceptable diluent comprised in the effervescent formulation of the present invention is maltodextrin.

In other words, one characteristic feature of the effervescent formulations of the present invention is that
- the formulations comprise alendronate in the range of 0.1 to 30 % by weight, maltodextrin as diluent, at least one effervescent couple in the range of 50 to 90 % by weight and optionally at least one other pharmaceutically acceptable excipient and
- alendronate :maltodextrin ratio is in the range of 1.1 to 0.9 by weight.

The amount of maltodextrin comprised in the effervescent formulation of the present invention is in the range of 0.1-30% by weight, preferably in the range of 1-20% by weight. In other words, one characteristic feature of the effervescent formulations of the present invention is that
- the formulations comprise alendronate in the range of 0.1 to 30 % by weight, maltodextrin in the range of 0.1-30% by weight as diluent, at least one effervescent couple in the range of 50 to 90 % by weight and optionally at least one other pharmaceutically acceptable excipient and
- alendronate:maltodextrin ratio is 1:1 by weight.

The acidic agent mentioned herein can be selected from a group comprising acetic acid, citric acid, lactic acid, malic acid, phosphoric acid, propionic acid, tartaric acid or pharmaceutically acceptable hydrates or anhydrates thereof or a combination thereof.

The acidic agent comprised in the effervescent formulation of the present invention is preferably citric acid or a pharmaceutically acceptable hydrate or anhydrate thereof.

The basic agent mentioned herein can be selected from a group comprising potassium carbonate, potassium bicarbonate, potassium citrate, potassium hydroxide, sodium carbonate, sodium hydrogen carbonate, sodium hydrogen citrate or a combination thereof.

The other excipients that can be used in the effervescent formulations of the present invention can be binder, lubricant, glidant, diluent, disintegrant, flavouring agent, sweetener, colouring agent, surfactant, antifoaming agent, viscosity agent, stabilizing agent or the combinations thereof.

The binder mentioned herein can be selected from starches such as potato starch, corn starch, wheat starch; sugars such as sucrose, glucose, dextrose, lactose, maltodextrin; natural and synthetic gums; gelatin; cellulose derivatives such as microcrystalline cellulose, HPC, HEC, HPMC, carboxymethylcellulose, methylcellulose, ethylcellulose; polyvinylpyrrolidone (povidone); polyethylene glycol (PEG); waxes; calcium carbonate; calcium phosphate; alcohols such as sorbitol, xylitol, mannitol and water or a combination thereof.

The binder comprised in the effervescent formulation of the present invention is preferably sorbitol.

The amount of the binder comprised in the effervescent formulation of the present invention is in the range of 0.01-10% by weight.

The lubricant mentioned herein can be selected from sodium lauryl sulphate, sodium benzoate, sodium chloride, sodium acetate, sodium fumarate, carbowax 4000, L-leucine (17), PEG or a combination thereof.

The glidant mentioned herein can be selected from talc, magnesium stearate, stearic acid, sodium stearyl fumarate, polyoxyethylene glycol, leucine, alanine, glycine, sodium benzoate, sodium acetate, fumaric acid or a combination thereof.

The disintegrant mentioned herein can be selected from starches such as potato starch, corn starch, wheat starch, pregelatinized starch, sodium starch glycolate; cellulose derivatives such as croscarmellose sodium or microcrystalline cellulose; polyvinylpyrrolidone; crospovidone; alginic acid and its salts; chyles such as xanthan gum and veegum; ion exchange resins or a combination thereof.

The flavouring agent mentioned herein can be selected from natural aroma oils (such as peppermint oil, oil of wintergreen, clove bud oil, parsley oil, eucalyptus oil, lemon oil, orange oil), menthol, menthane, anethole, methyl salicylate, eucalyptol, cinnamon, 1- methyl acetate, sage, eugenol, oxanon, alpha-irisone, marjoram, lemon, orange, blackberry, propenyl guaetol acetyl, cinnamon, vanilla, timole, linalol, cinnamaldehyde glycerol acetal, N-substituted p-menthane-3-carboxamide, 3,1-methoxy propane 1.2-diol or a combination thereof.

The sweetener mentioned herein can be selected from sucralose, sucrose, fructose, glucose, galactose, xylose, dextrose, laevulose, lactose, maltose, maltodextrin, mannitol, maltitol, maltol, sorbitol, xylitol, erythritol, lactitol, isomalt, corn syrup, saccharine, saccharine salts, acesulphame potassium, aspartame, D-tryptophan, monoammonium glycyrrhizinate, neohesperidin dihydrochalcone, thaumatin, neotame, alitame, stevioside and cyclamates or a combination thereof.

The colouring agent mentioned herein can be selected from carotenoids and chlorophyll or a combination thereof.

The surfactant mentioned herein can be selected from sodium lauryl sulphate and magnesium lauryl sulphate or a combination thereof.

The antifoaming agent mentioned herein can be selected from simethicone emulsion and dimethyl siloxane, silicone oil or a combination thereof.

The viscosity agent mentioned herein can be selected from carboxymethyl cellulose, methyl cellulose, xanthan gum, gummi tragacanthae, gum arabic, aerosil 200, colloidon, agar-agar, bentonite, hydroxyethyl cellulose or a combination thereof.

The stabilizing agent and/or agents mentioned herein are selected from antioxidants, chelating agents, alkalinizing agents and photo protective agents.

The antioxidants can be selected from substances such as butylated hydroxyanisole (BHA), sodium ascorbate, butylated hydroxytoluene (BHT), sodium sulphite, gallates (such as propyl gallate), tocopherol, citric acid, malic acid, ascorbic acid, acetylcysteine, fumaric acid, lecithin, ascorbyl palmitate, ethylenediamine tetraacetate or a combination thereof.

The chelating agents can be selected from disodium EDTA, edetic acid, citric acid, sodium citrate, potassium citrate or a combination thereof.

The alkalizing agents can be selected from alkali metal salts such as sodium carbonate, sodium hydrogen carbonate, sodium hydroxide, sodium silicate, disodium hydrogen ortophosphate, sodium aluminate; earth alkali metal salts such as calcium carbonate, calcium hydroxide, dibasic calcium phosphate, tribasic calcium phosphate, calcium sulphate, calcium acetate, calcium gluconate, calcium glycerophosphate, magnesium carbonate, magnesium hydroxide, magnesium sulphate, magnesium acetate, magnesium silicate, magnesium aluminate and organic compounds such as primary, secondary and tertiary amines, cyclic amines, N-N'-dibenzylethylenediamine, diethanoleamine, ethylenediamine, meglumine, monosodium glutamate, polyacryline sodium, sodium alginate or a combination thereof.

The photoprotective agents can be selected from metal oxides such as titanium oxide, iron oxide or zinc oxide or a combination thereof. Disclosed is also an effervescent formulation comprising
- alendronate and/or a pharmaceutically acceptable derivative thereof in the range of 0.1-30 % by weight,
- effervescent couple in the range of 50-90% by weight
- binder in the range of 0.01-10% by weight
- diluent in the range of 0.1-30% by weight
- other excipients in the range of 0.1-50 % by weight.

The formulation of the present invention can be optionally combined with the other active agent.

The term "other active agent" mentioned herein refers to various vitamins and/or minerals required for human body.

The dosage forms can be taken separately, simultaneously and sequently for combined therapy, as well as taken as combination of alendronate with the other active agent or agents in a single dosage form.

The other active agent or agents that can be used with alendronate in combined therapy can be minerals such as calcium, potassium, magnesium, iron, sodium, zinc or salts thereof such as carbonate, sulphate; vitamins such as vitamin A, B vitamins such as B₁, B₁₂, B₆ and/or folic acid; vitamin C, vitamin D and its analogs (such as Vitamin D2 (ergocalciferol), vitamin D3 (cholecalciferol)), vitamin E.

One or two of the other active agents listed above can be combined with alendronate in combined therapy. In other words, the present invention refers to binary and ternary combinations of alendronate with the other active agents specified above.

The second active agent which is preferred is vitamin D and it analogs (such as Vitamin D2 (ergocalciferol), vitamin D3 (cholecalciferol)) and/or calcium.

The other active agent or agents that can be used in combined therapy can be produced with alendronate and by the same production method, as well as can be prepared as combined treatment package by producing the active agent formulations separately and combining them afterwards.

The formulations of the present invention can be produced with any methods in the prior art. Dry granulation, dry blending, direct compression, wet granulation can be listed among these methods.

The formulation of the present invention can be used in treatment of osteoporosis; in order to reduce risk of bone fracture in women including spine bone and hipbone; in order to reduce risk of bone fracture in men; in treatment of diseases such as idiopathic osteoporosis; osteoporosis induced various diseases; steroid and glucocorticoid-induced osteoporosis ; osteopenia, osteomalacia, osteogenesis imperfecta, osteochondrodysplasia, sudeck atrophy, rheumatoid arthritis, Paget's disease, bone metastasis of malignant tumors, hypercalcaemia or hyperthyroidism; and particularly in adolescence, pregnant or nursing women in premenopausal and postmenopausal period in order to preserve bone health as nutrition reinforcement.

The examples required in order to render the formulation of the present invention more comprehensible are given below. Yet, the present invention should not be limited to these examples.

### DISCLOSED EXAMPLES

### Example 1

Granulation solution is prepared by mixing the binder and the diluent. Alendronate and effervescent couple are mixed in fluid bed and granulated with the granulation solution prepared. The granules obtained are mixed with the lubricant and the other excipients. The formulation prepared can be packed in the form of powder or granule and can also be compressed in tablet form.

| **Component** | **Amount (% by weight)** |
|---|---|
| Alendronate sodium trihydrate | 4,6 |
| Effervescent couple | 75 |
| Binder | 2 |
| Diluent | 4,4 |
| Other excipients | 14 |
| **Total** | **100** |

### Example 2

| **Component** | **Amount (% by weight)** |
|---|---|
| Alendronate sodium trihydrate | 3,5 |
| Effervescent couple | 80 |
| Binder | 1,6 |
| Diluent | 3,5 |
| Other excipients | 11,4 |
| **Total** | **100** |

## Claims

1. An effervescent formulation comprising alendronate, a pharmaceutically acceptable diluent and optionally at least one other excipient, **characterized in that** the diluent used in the formulation is maltodextrin and the ratio of alendronate to maltodextrin used in the formulation is in the range of 1.1 to 0.9 by weight.

2. The effervescent formulation according to claim 1, **characterized in that** alendronate comprised in said formulation is in sodium salt form.

3. The effervescent formulation according to claim 2, **characterized in that** alendronate comprised in said formulation is in hydrate form.

4. The effervescent formulation according to claim 3, **characterized in that** alendronate comprised in said formulation in trihydrate form.

5. The effervescent formulation according to claim 4, **characterized in that** said formulation comprises alendronate sodium trihydrate.

6. The effervescent formulation according to claim 1, **characterized in that** the amount of alendronate comprised in said formulation is in the range of 0.1-30% by weight.

7. The effervescent formulation according to claim 6, **characterized in that** the amount of alendronate comprised in said formulation is in the range of 0.1-20% by weight.

8. The effervescent formulation according to claim 1, **characterized in that** said formulation comprises at least one pharmaceutically acceptable effervescent couple and optionally at least one other excipient.

9. The effervescent formulation according to claim 8, **characterized in that** the effervescent couple comprised in said formulation is composed of acidic agent and basic agent.

10. The effervescent formulation according to claim 8, **characterized in that** the excipients are selected from a group comprising binder, lubricant, glidant, diluent, disintegrant, flavouring agent, sweetener, colouring agent, surfactant, antifoaming agent, viscosity agent, stabilizing agent or a combination thereof.

11. The effervescent formulation according to claim 10, **characterized in that**
• said formulations comprise alendronate in the range of 0.1 to 30 % by weight, maltodextrin as diluent, at least one effervescent couple in the range of 50 to 90% by weight and optionally at least one other pharmaceutically acceptable excipient and
• alendronate:maltodextrin ratio in said formulations is in the range of 1.1 to 0.9 by weight.

12. The effervescent formulation according to claim 11, **characterized in that** said formulations comprise maltodextrin in the range of 0.1 to 30% by weight, preferably in the range of 1-20% by weight.

13. The effervescent formulation according to any preceding claims, **characterized in that** said formulations comprise at least one other active agent selected from a group comprising minerals such as calcium, potassium, magnesium, iron, sodium, zinc or salts thereof such as carbonate, sulphate; vitamins such as vitamin A, B vitamins such as B₁, B₁₂, B₆ and/or folic acid; vitamin C, vitamin D such as vitamin D2 (ergocalciferol) or vitamin D3 (cholecalciferol), and vitamin E.

## Patentansprüche

1. Eine übersprudelnde Formulierung bestehend aus Alendronat, einem pharmazeutisch annehmbaren Verdünner und eventuell mindestens einem anderen Arzneistoffträger, **dadurch gekennzeichnet, daß** der Verdünner, der in Formulierung verwendet wird, Maltodextrin ist und das Verhältnis von Alendronat zum Maltodextrin verwendet in Formulierung ist im Bereich von 1.1 bis 0.9 nach Gewicht.

2. Die übersprudelnde Formulierung nach Anspruch 1, **dadurch gekennzeichnet, daß** Alendronat beinhaltend in besagter Formulierung ist in Form von Natriumsalz.

3. Die übersprudelnde Formulierung nach Anspruch 2, **dadurch gekennzeichnet, daß** Alendronat beinhaltend in besagter Formulierung ist in Form von Hydrat.

4. Die übersprudelnde Formulierung nach Anspruch 3, **dadurch gekennzeichnet, daß** Alendronat beinhaltend in besagter Form ist in Form von Trihydrat.

5. Die übersprudelnde Formulierung nach Anspruch 4, **dadurch gekennzeichnet, daß** besagte Formulierung beinhaltend Alendronat-Natrium-Trihydrat.

6. Die übersprudelnde Formulierung nach Anspruch 1, **dadurch gekennzeichnet, daß** die Menge von Alendronat beinhaltend in besagter Formulierung ist im Bereich von 0.1-30 % nach Gewicht.

7. Die übersprudelnde Formulierung nach Anspruch 6, **dadurch gekennzeichnet, daß** die Menge von Alendronat beinhaltend in besagter Formulierung ist im Bereich von 0.1-20 % nach Gewicht.

8. Die übersprudelnde Formulierung nach Anspruch 1, **dadurch gekennzeichnet, daß** besagte Formulierung bestehend aus mindestens einem pharmazeutisch annehm-baren übersprudelnden Paar und eventuell mindestens einem anderen Arzneistoffträger.

9. Die übersprudelnde Formulierung nach Anspruch 8, **dadurch gekennzeichnet, daß** übersprudelndes Paar beinhaltend in besagter Formulierung bestehend aus Säuremittel und Alkalimittel.

10. Die übersprudelnde Formulierung nach Anspruch 8, **dadurch gekennzeichnet, daß** Arzneistoffträger ausgewählt sind von einer Gruppe bestehend aus Verbinder, Schmiermittel, Gleitstoff, Verdünner, Sprengmittel, Aromastoff, Süßstoff, Farbstoff, Benetzungsmittel, Schaumverhütungsmittel, Viskositätsmittel, Stabilisierungsmittel oder einer Kombination von denen.

11. Die übersprudelnde Formulierung nach Anspruch 10, **dadurch gekennzeichnet, daß**
• besagte Formulierungen beinhalten Alendronat im Bereich von 0,1 bis 30 % nach Gewicht, Maltodextrin als Verdünner, mindestens ein übersprudelndes Paar im Bereich von 50 bis 90 % nach Gewicht und eventuell mindestens einen anderen
pharmazeutisch annehmbaren Arzneistoffträger und
• Alendronat: Maltodextrin Verhältnis in besagten Formulierungen ist im Bereich von 1.1 bis 0.9 nach Gewicht.

12. Die übersprudelnde Formulierung nach Anspruch 11, **dadurch gekennzeichnet, daß** besagte Formulierungen beinhalten Maltodextrin im Bereich von 0.1 bis 30 % nach Gewicht, bevorzugt im Bereich von 1-20 % nach Gewicht.

13. Die übersprudelnde Formulierung nach den vorhergehenden Ansprüche, **dadurch gekennzeichnet, daß** besagte Formulierungen beinhalten mindestens ein anderes aktives Mittel ausgewählt von einer Gruppe bestehend aus Minerale wie Calcium, Kalium, Magnesium, Eisen, Natrium, Zink oder deren Salze wie Karbonate, Sulfate; Vitamine wie Vitamin A, B Vitamine wie B₁,B₁₂ B₆ und/oder Folsäure; Vitamin C, Vitamin D wie Vitamin D₂ (Ergocalciferol) oder Vitamin D₃ (Cholecalciferol), und Vitamin E.

## Revendications

1. Formulation effervescente comprenant de l'alendronate, un diluant pharmaceutiquement acceptable et éventuellement au moins un autre excipient, **caractérisé en ce que** le diluant utilisé dans la formulation est la maltodextrine et le rapport de l'alendronate à la maltodextrine utilisé dans la formulation est compris entre 1,1 et 0,9 en poids.

2. Formulation effervescente selon la revendication 1, **caractérisée en ce que** l'alendronate compris dans ladite formulation est sous forme de sel de sodium.

3. Formulation effervescente selon la revendication 2, **caractérisée en ce que** l'alendronate compris dans ladite formulation est sous forme hydratée.

4. Formulation effervescente selon la revendication 3, **caractérisée en ce que** l'alendronate comprend dans ladite formulation sous forme trihydrate.

5. Formulation effervescente selon la revendication 4, **caractérisée en ce que** ladite formulation comprend un alendronate de trihydrate de sodium.

6. Formulation effervescente selon la revendication 1, **caractérisée en ce que** la quantité d'alendronate comprise dans ladite formulation est dans la plage de 0,1 à 30% en poids.

7. Formulation effervescente selon la revendication 6, **caractérisée en ce que** la quantité d'alendronate comprise dans ladite formulation est dans la plage de 0,1 à 20% en poids.

8. Formulation effervescente selon la revendication 1, **caractérisée en ce que** ladite formulation comprend au moins un couple effervescent pharmaceutiquement acceptable et éventuellement au moins un autre excipient.

9. Formulation effervescente selon la revendication 8, **caractérisée en ce que** le couple effervescent compris dans ladite formulation est composé d'un agent acide et d'un agent basique.

10. Formulation effervescente selon la revendication 8, **caractérisée en ce que** les excipients sont choisis parmi un groupe comprenant liant, lubrifiant, glissement, diluant, désintégrant, agent aromatisant, édulcorant, agent colorant, tensioactif, agent antimousse, agent de viscosité, agent stabilisant ou une combinaison de celui-ci.

11. Formulation effervescente selon la revendication 10, **caractérisée en ce que**
• lesdites formulations comprennent de l'alendronate dans la plage de 0,1 à 30% en poids, la maltodextrine en tant que diluant, au moins un couple effervescent dans la plage de 50 à 90% en poids et éventuellement au moins un autre excipient pharmaceutiquement acceptable et
• alendronate: maltodextrine dans lesdites formulations est compris entre 1,1 et 0,9 en poids.

12. Formulation effervescente selon la revendication 11, **caractérisée en ce que** lesdites formulations comprennent de la maltodextrine dans la plage de 0,1 à 30% en poids, de préférence dans la plage de 1 à 20% en poids.

13. Formulation effervescente selon l'une quelconque des revendications précédentes, **caractérisée en ce que** lesdites formulations comprennent au moins un autre agent actif choisi parmi un groupe comprenant des minéraux tels que le calcium, le potassium, le magnésium, le fer, le sodium, le zinc ou leurs sels tels que le carbonate, le sulfate; des vitamines telles que la vitamine A, les vitamines B telles que B₁, B₁₂ B₆ et / ou l'acide folique; vitamine C, vitamine D comme la vitamine D₂ (ergocalciferol) ou vitamine D₃ (cholécalciferol) et vitamine E.
